Europäisches Patentamt

European Patent Office

Office européen des brevets

(19)

(11) Publication number: **0 167 411**
**B1**

(12) **EUROPEAN PATENT SPECIFICATION**

(45) Date of publication of patent specification: **09.01.91**

(21) Application number: **85304829.6**

(22) Date of filing: **05.07.85**

(51) Int. Cl.⁵: **C 12 P 13/22** // (C12P13/22, C12R1:645)

(54) **Microbiological preparation of L-Phenylalanine.**

(30) Priority: **05.07.84 JP 139387/84**
**27.05.85 JP 113823/85**

(43) Date of publication of application:
**08.01.86 Bulletin 86/02**

(45) Publication of the grant of the patent:
**09.01.91 Bulletin 91/02**

(84) Designated Contracting States:
**CH DE FR GB IT LI NL**

(56) References cited:
CHEMICAL ABSTRACTS, vol. 78, no. 19, 14th
May 1973, page 227, abstract no. 121414g,
Columbus, Ohio, US; P.W. PERRIN et al.:
"Metabolism of aromatic acids by polyporus
hispidus"
CHEMICAL ABSTRACTS, vol. 80, no. 9, 4th
March 1974, page 248, abstract no. 46542h,
Columbus, Ohio, US
CHEMICAL ABSTRACTS, vol. 88, no. 5, 30th
January 1978, page 360, abstract no. 35841u,
Columbus, Ohio, US

(73) Proprietor: **MITSUBISHI KASEI CORPORATION**
**5-2, Marunouchi 2-chome Chiyoda-ku**
**Tokyo 100 (JP)**

(72) Inventor: **Kamata, Akira**
**Aobadai Daiichi Apt. 142 6, Tsutsujigaoka 5-chome**
**Midori-ku Yokohama-shi Kanagawa (JP)**
Inventor: **Mikawa, Takashi**
**17-4-3, Nishi Hashimoto 1-chome**
**Sagamihara-shi Kanagawam (JP)**
Inventor: **Imada, Yukio**
**10-12, Narusedai 2-chome**
**Machida-shi Tokyo (JP)**

(74) Representative: **Diamond, Bryan Clive et al**
**Gee & Co., Chancery House, Chancery Lane**
**London WC2A 1QU (GB)**

(56) References cited:
APPLIED AND ENVIRONMENTAL
MICROBIOLOGY, vol. 42, no. 5, November 1981,
pages 773-778; S. YAMADA et al.: "Production
of L-phenylalanine from trans-cinnamic acid
with rhodotorula glutinis containing L-phenylalanine ammonia-lyase activity"

# EP  0 167 411  B1

(56) References cited:
CHEMICAL ABSTRACTS, vol. 97, no. 7, 16th
August 1982, page 166, abstract no. 50563j,
Columbus, Ohio, US; L.A. SIKORA et al.:
"Regulation of L-phenylalanine ammonia-lyase
by L-phenylalanine and nitrogen in Neurospora
crassa"

CHEMICAL ABSTRACTS, vol. 98, no. 13, 28th
March 1983, pages 359,360, abstract no.
104369s, Columbus, Ohio, US; Z. ZOUCHOVA et
al.: "Metabolism of aromatic acids in the
antibiotic-producing basidiomycete
Oudemansiella mucida"

## Description

The present invention relates to a process for the preparation of L-phenylalanine, more particularly, to a process for the preparation of L-phenylalanine by reacting trans-cinnamic acid with ammonia in the presence of a phenylalanine ammonia-lyase.

It is known to prepare L-phenylalanine by reacting trans-cinnamic acid with ammonia in the presence of a phenylalanine ammonia-lyase produced by a microorganism.

Examples of such microorganism include *Sporoboromyces roseus* 227 (FERM P-3852), *Rhodotorula gracilis* (IFO 0559), *Rhodotorula marina* (IFO 0879), *Fusarium oxysporum* (ATCC 20395), etc. as described in, for example, British Patent No. 1,489,468 and Japanese Patent Application (OPI) No. 26197/1981. However, it is said that the activity of phenylalanine ammonia-lyases obtained from these microorganisms is relatively low and decays rapidly.

Thus, it is desirable to discover microorganisms producing a phenylalanine ammonia-lyase having a high and persistent activity.

An object of the present invention is to provide a process for the preparation of L-phenylalanine by reacting cinnamic acid with ammonia in the presence of a phenylalanine ammonia-lyase produced by such microorganisms.

It has now been found that the above-described object is attained by the use of microorganisms belonging to species of specified genera. Therefore, the present invention provides a process for the preparation of L-phenylalanine, comprising reacting cinnamic acid with ammonia in the presence of a phenylalanine ammonia-lyase obtained from a microorganism and belonging to species of *Cladosporium, Pholiota, Hebeloma, Coprinus* or *Lyophyllum* having an ability of producing L-phenylalanine from trans-cinnamic acid and ammonia.

The phenylalanine ammonia-lyase used in the present invention is obtained from a microorganism which belongs to species of *Cladosporium, Pholiota, Hebeloma, Coprinus* or *Lyophyllum* and has an ability of producing L-phenylalanine from trans-cinnamic acid and ammonia.

Examples of such microorganism include *Cladosporium cladosporioides* C-5056 (FERM P-7704), *Pholiota highlandensis* MCI 2037 (FERM P-8242), *Hebeloma vinosophyllum* MCI 2038 (FERM P-8243), *Coprinus stercorarius* MCI 2035 (FERM P-8241) and *Lyophyllum tylicolor* MCI 2034 (FERM P-8240).

The microbiological properties of these fungi and taxonomic criteria for the identification of them are as follows:

A. *Cladosporium cladosporioides* C-5056:
(1) Microscopic properties:
    a) Characteristics on a malt agar medium (MA):
    Colonies grow moderately by culture for one week, up to 1.5 cm diameter. The tint of colonies is olive green; the reverse is dark green to black colored. Vegetative hyphae are first colorless and afterwards brownish, their breadth amounting to 12 μm; aerial hyphae grow well. Conidiophores grow from vegetative hyphae; usually they do not branch; their length is 25 to 380 μm and their breadth is 2.0 to 6.0 μm; they form conidial heads which are smooth, have septal walls, are brown colored and are branched dendriticly. Conidia are blastoconidia, which are egg-shaped, lemon-shaped, oval or cylindrical, pale brown, smooth single cells of 2 to 11 μm×2 to 4 μm; the basal conidia are cylindrical and sometimes composed of 2 to 3 cells. Sexual generative organs were not observed.
    b) Characteristics on a potato-dextrose agar medium (PDA):
    Same as the properties on a malt agar medium.

(2) Physiological properties:
    Growing temperature: 10 to 30°C
    Optimum growing temperature: 20 to 26°C
    Growing pH: 2 to 10
    Optimum growing pH: 4 to 7.

(3) Taxonomic consideration:
    This strain (C-5056), forms colored colonies of olive green to dark green. Conidium ontogeny blastoconidia-type, and so the strain belongs to the genus *Cladosporium* in *Deuteromycotina*.
    According to Ellis: "Dematiaceous Hyphomycetes" (1971, 1976), 43 species are described under the genus *Cladosporium*. These species are identified by difference in tint of colonies; specificity of host plants; size, shape, surface structure and cell numbers of conidia; and the presence or absence of perfect stage.
    The cultural and morphological characteristics of this strain (C-5056) well agreed with those of *Cladosporium cladosporioides* described by Ellis (1971).
    Therefore, the strain (C-5056) was identified as *Cladosporium cladosporioides*.

B. *Pholiota Highlandensis* MCI 2037:
    Caps have each a breadth of 2 to 6 cm and many of them are rising at the central, though some of them are not rising. They are colored near the central chestnut brown to orangish chestnut brown or reddish brown, and at the base pale yellow to dirty yellow with the periphery paled. They are highly viscous and lustrous. There are reddish brown danders adhered to the peripheral portion of the caps.
    Lamellae are straight, but curve by aging. They are thick and considerably broad. They are colored first pale and afterwards pale grayish brown to brown.
    Stems are each of a size of 1.5 to 5 cm×0.3 to 0.5 cm. They are thick in the upper part and slender in the lower part. They are coarse, hard, elastic and hollow, and have fibrous scales.
    Spores are egg-shaped, smooth, and yellowish brown to brown colored, and have each a size of 5.5 to 6.5 μm×4 to 4.5 μm. The spore particles are dirty grayish brown colored.

Basidia are each of a size of 25 to 32 μm×7 to 8 μm. They are subcylindrical to clubbish, 4 spore-forming and colorless to yellow colored.

Pleuro-cystidia are each of a size of 56 to 63 μm×12 to 17 μm, partly spindle-shaped and have bluntly pointed ends. Cheilo-cystidia are each of a size of 48 to 54 μm×11 to 13 μm and clubbish to somewhat spindle-shaped. Cystidia of stems are each of a size of 45 to 88 μm×7 to 16 μm and cylindrical, clubbish or partly spindle-shaped.

The outer skin of the upper layer of caps of this strain (MCI 2037) consists of thin linear hyphae provided with rostriform projections, and that of the lower layer is racemose. Spore prints are dirty grayish brown colored, and the spores have germ pores. Thus, the strain belongs to *Strophariaceae*.

This strain has danders on the surface of caps and is highly viscous, and its spores are smooth and have germ pores. It always has cheilo-cystidia. From these characteristics it well agrees with the genus *Pholiota* as described in Imazeki & Hongo: "Japan Fungi Heliochrome Picture Book", p. 62 (1972).

The morphological characteristics, such as caps, stems, spores, cysitida, etc., of this strain well agree with the properties of *Pholiota highlandensis* described in Seiya Ito: *Japan Fungi Bulletin*, Vol. 2, No. 5, p. 352 (1961) and Imazeki & Hongo: "Japan Fungi Heliochrome Picture Book" p. 63 (1972).

C. *Hebeloma Vinosophyllum* MCI 2038:

Caps are hemispheric mountain-shaped or a little spread conical mountain-shaped and of various sizes, 1—6—11 cm. They are colored creamy to celadonish milky white. The central part is brownish. There are many variations in their color. They vary from almost white to yellow brown or yellow ochre. As dried, they become lustrous and covered with smooth and viscous mycelial membrane which is first colored similar to white. At young age, their periphery coils inside. Their fruit body is rather thick, having a thickness of 3 mm to 7 mm, colored white or creamy, and has a bitter taste and no odor. Lamellae are of a moderate breadth of 1 to 11 mm, somewhat thick, and first straight and afterwards curved or somewhat separated. They are colored first light pinky violet or pale violet brown and afterwards reddish violet flesh brown, violet brown or maroon. Stems are each of a size of 2.5 to 9 cm×3 to 11 mm, and of about 1.6 cm at the basal thick portion. Usually, the basal portion is partly spindle-shaped, particularly of young age. Their surface is same colored as caps. They are colored creamy to creamy brown and have dirty lines of yellow brown color. They are first not hollow but afterwards hollow. They are not viscous. They have white hyphae at the basal portion, and their upper portion is white powdery.

Spores are rough surfaced and verrucous, and broad apricot stone-shaped. They are each of a size of 9 to 11 μm×6.5 to 7 μm. Spore prints are flesh brown colored.

Basidia are 4 spores forming and each of a size of 30 to 40 μm×8 to 9 μm. Very many cheilo-cystidia grow thick. They are needle-like spindle-shaped, pin-shaped or long neck spindle-shaped, each of a size fo 37 to 60 μm×7 to 18 μm and colorless. Their pellicle is easily broken. There are no pleuro-cystidia. Hyphae have clamp connections. Hyphae on the viscous surface layer of caps have each a thickness of 5 to 7 μm and many clamp connections.

Spore prints of this strain (MCI 2038) are colored flesh brown. Spores are rough surfaced and verrucous, and have no germ pores. Therefore, the strain belongs to *Cortinariaceae*.

This strain has viscosity on the surface of caps, many cheilo-cystidia and no pleuro-cystidia. Thus, the strain agrees well with the genus Hebeloma defined in Imazeki & Hongo: "Japan Fungi Heliochrome Picture Book" (1972).

This strain, being colored crimson brown in its lamellae and spore prints, can easily be differentiated from other species.

D. *Coprinus Stercorarius* MCI 2035:

Caps are each of a height of 1 to 2 cm and of a diameter of 5 to 10 mm. Their surface is covered with pale gray to grayish brown or grayish black powder, especially at the top portion. Although any stripes cannot be seen at first because of such powder, the powder falls off as the caps open, and fine and deep streaks appear from the peripheral portion.

The fruit body is coloured dark grayish, brown, thin and of no taste. Sometimes, it has an offensive odor.

Lamellae are colored first white and afterwards black. They are thin and appear thick. Their breadth is 1.5 to 2 mm. Their periphery is white powdery.

Stems are colored dirty white to grayish white. Their thickness is same at their upper portion and lower portion. At young age, they are coated with something like fine hair or soft down, which is colored pale yellowish brown or pale grayish brown.

Stems extend long and slenderly into the earth.

Spores are oval with blunt terminals. They are colored dark reddish brown, each of a size of 9 to 11 μm×5 to 6 μm and not flat. They have germ pores. Spore prints are colored black.

Basidia are 4 spores forming and each of a size of 15 to 30 μm×7 to 9 μm.

Cheilo-cystidia are spherical or sac-shaped, and pleuro-cystidia are long sac-shaped.

The powdery coating membrane of caps consists of branched linear mycelia provided with projections, and spherical sacs. The pellicle of the spherical sacs is easily broken. The spherical sacs are slightly verrucous and each of a diameter of 30 to 100 μm.

The coating membrane of stems consists of branched linear hyphae provided with many small projections, somewhat like coral, having a thickness of 2 to 10 μm.

This strain (MCI 2035), being colored black in its spore prints, having germ pores in its spores and having short basidia, belongs to the genus *Coprinus*.

This strain (MCI 2035) has radiate streaks at the peripheral portion of cap and so its cap is fan-shaped. The spores mature first at the lower portion and then at the upper portion of lamellae, whereby the pores begin to be liquefied at the matured portion and drop down like black colored ink. The strain has pleuro-cystidia.

These basic properties well agree with the definition of the genus *Coprinus* described in Imazeki & Hongo: "Japan Fungi Heliochrome Picture Book" p. 53 (1972).

Caps of this strain are, at young age, covered with powdery and granular coating membrane. The fruit body is of small size and is characterized by growing from a sclerotium. Thus, the characteristics of this strain well agree with those of *Coprinus stercorarius* as described in Seiya Ito: *Japan Fungi Bulletin*, Vol. 2, No. 5, p. 300 (1961) and in Imazeki & Hongo: "Japan Fungi Heliochrome Picture Book, continued" p. 58 (1972).

E. *Lyophyllum tylicolor* MCI 2034:

Caps grow each up to a diameter of 1.5 cm. They are first egg-shaped and afterwards hemispherical- to mountain-shaped, and their central portion is slightly convex. At young age, they are colored dark brown, their top portion is colored deeply and is almost black brown, and their lower portion is colored somewhat palely, being provided with similar white silky fibers. The opened caps are colored brown ochre, and their peripheral portion is pale and patterned with deep and pale stripes, with transparent feeling. At young age, a slight inner coating membrane is present at the edge.

The fruit body is, as it is wet, colored dark brown ochre, with transparent feeling. It has no taste and no odor.

Lamellae are thick and somewhat rough, consisting of 32 leaves. They are each of a wide breadth, curved straightly and somewhat triangle shaped. They are colored first grayish white to gray and afterwards pale dark ochre (dirty ochre). They give a somewhat powdery or waxy feeling. Their edge is somewhat blunt.

Stems are each of a size of 2 to 2.5 cm×2 to 2.5 mm, and their thickness is the same at the upper and lower portions. They are colored first powdery white and afterwards the same as caps. The upper portion is pale, and the lower portion is dark. They are coated with fine powdery fibers. They are hollow and the inside is colored dark brown.

Basidia are 4 spores forming (26 to 30 μm×8 to 9 μm). Both cheilo- and pleuro-cystidia are not present.

Spores are colorless, egg-shaped to broad oval, each of a size of 6.5 to 8 μm×4.5 to 5 μm, and have fine verrucous projections. Each spore contains one drop of oil. They are not amyloid.

Hyphae lack clamp connections.

Spore prints of this strain (MCI 2034) are colored white and do not become colored deeply or brightly. The strain belongs to *Tricholomataceae* since its spores are not amyloid and have no germ pores.

The fruit body of this strain (MCI 2034) is colored dark brown to black brown to ochre brown, and the basidia of the strain involve granules easily stained by acetocarmine (carminophilous granulation). These basic characteristics agree with the definition of the genus *Lyophyllum* as described in Imazeki & Hongo: "Japan Fungi Heliochrome Picture Book, continued" p. 17.

Phenylalanine ammonia-lyase may be used in the form of a culture broth of the above-mentioned microorganism, etc., cells obtained from the culture broth by centrifugation, and washed and dried or cells obtained by further treatments of the cells, and also in the form of a solution of enzyme, immobilized enzyme, or the like.

There is no special limitation on the medium used incubation of the culture. Various carbohydrates, organic acids, etc. can be used as carbon source, and as nitrogen source, organic ammonium salts, inorganic ammonium salts, urea, etc. can be used. Further, various phosphates, sulfates and the like may be used as inorganic material, if desired, and various organic nutrients may also be added to the medium.

Cultures are incubated under aerobic condition, usually for about 6 hours to 5 days with regard to *Cladosporium cladosporioides* C-5056 and usually for about 1 day to 10 days with regard to *Pholiota highlandensis* MCI 2037, *Hebeloma vinosophyllum* MCI 2038, *Coprinus stercorarius* MCI 2035 and *Lyophyllum tylicolor* MCI 2034. The pH of medium is selected from 3 to 10, and the incubation temperature is selected from about 20 to 40°C.

Examples of ammonia used in the above described reaction include aqueous ammonia and ammonia precursors, e.g., ammonium salts such as ammonium sulfate, ammonium chloride, ammonium acetate, and the like. The reaction can be carried out by a batch process, semi-batch process or continuous process. In the reaction, a transcinnamic acid concentration of about 0.1 to 5 w/v% and an ammonia concentration of about 1 to 15 w/v% are usually employed. The reaction temperature is usually selected from the range of about 10 to 60°C, and the pH is usually selected from the range of about 8 to 11.5. Although the reaction time varies depending on the reaction conditions and others, it is usually selected from the range of about several hours to 4 days in the batch process. After completion of the reaction, isolation of L-phenylalanine from the reaction products and purification of the isolated L-phenylalanine may be carried out according to ordinary methods.

Hereinafter, the present invention is explained further in detail by some Examples.

Example 1

*Cladosporium cladosporioides* C-5056 (FERM P-7704) was inoculated with 1 loopful to a medium having the following composition (pH 6.0, 100 ml in a conical flask of 500 ml volume) and incubated at 26°C for 3 days on a rotary shaker.

Composition of medium (in 100 ml)

| | |
|---|---|
| Peptone | 3.0 g |
| Yeast extract | 0.5 g |
| NaCl | 0.5 g. |

From the culture medium (100 ml) thus obtained, cells were collected by centrifugation. Next, 3.0 g of trans-cinnamic acid was dissolved in 55 ml of 28% aqueous ammonia and, after adjusted to pH of 10.0 with HCl, 10.0 g of the above wet cells was suspended in the solution. By adding water, the cell suspension was diluted to 100 ml volume, and then the reaction was carried out at 30°C (for 24 hours). After the reaction, cells were removed by centrifugation. Then, it was determined that 0.4 g of L-phenylalanine was deposited in the reaction mixture.

The analysis was carried out by high-pressure liquid chromatography, using Zorbax ODS, as a column, mixture consisting of 600 ml of water, 400 ml of methanol, 0.5 ml of phosphoric acid and 50 mg of sodium 1-pentanesulfonate, elution buffer.

Example 2

The culture was incubated in the same manner as in Example 1, except that a medium having a different composition as described hereinafter was used.

Composition of medium (in 100 ml)

| | |
|---|---|
| Peptone | 3.0 g |
| Yeast extract | 0.5 g |
| NaCl | 0.5 g |
| Glucose | 0.5 g |
| L-Phenylalanine | 0.3 g. |

With the culture broth obtained, the reaction was carried out under the same conditions as Example 1. After 24 hours, cells were removed by centrifugation and the amount of L-phenylalanine in the supernatant was determined. It was determined that 0.6 g of L-phenylalanine was produced (the first reaction).

Next, after washing the cells, the reaction was carried out in the same manner as Example 1, with a newly added mixture for the reaction, using the cells again. After the reaction, cells were removed by centrifugation. It was determined that 0.4 g of L-phenylalanine was produced in the supernatant (the second time).

Comparative Example 1

The reaction was carried out twice in the same manner as Example 2, except that the strain was replaced by Rhodotorula glutinis (which is now classified into Rhodosporium toruloides).

The amount of L-phenylalanine produced was 0.12 g the first time, and 0.01 g the second time.

Example 3

Using 50 ml of medium having the composition as described below, contained in a ribbed conical flask of 200 ml volume, Lyophyllum tylicolor MCI 2034 (FERM P-8240) was incubated at 26°C for 6 days:

Composition of medium (pH 6.0)

| | |
|---|---|
| Glucose | 5 g |
| Peptone | 5 g |
| Yeast extract | 5 g |
| Corn steep liquor | 5 g |
| L-Phenylalanine | 3 g |
| Water | 1,000 ml. |

After the incubation, cells were collected from the culture broth by centrifugation. The cells were washed once with 0.8% aqueous NaCl solution and then dissolved in 27 ml of 28% aqueous ammonia containing 1.5 g of trans-cinnamic acid dissolved therein. The solution was adjusted to pH 10 with hydrochloric acid and then diluted to a volume of 50 ml with water. Next, the reaction was carried out at 30°C for 24 hours. It was recognized that 0.23 g of L-phenylalanine was produced in the reaction mixture.

After washing the cells, the reaction was carried out in the same manner as described above, with a newly added mixture for the reaction, using the cells again. In the resulting reaction mixture, 0.16 g of L-phenylalanine was produced.

Example 4

Incubation of culture and reaction were carried out in the same manner as Example 3, except that the strain was replaced by Coprinus stercorarius MCI 2035 (FERM P-8241). In the resulting reaction mixture, 0.14 g of L-phenylalanine was produced. Further, using the recovered cells again the reaction was repeated in the same manner as Example 3, whereby 0.10 g of L-phenylalanine was produced.

Example 5

Incubation of culture and reaction were carried out in the same manner as Example 3, except that the strain was replaced by Hebeloma vinosophyllum MCI 2038 (FERM P-8243) and the incubation was continued for 7 days. In the resulting reaction mixture, 0.17 g of L-phenylalanine was produced. As the reaction was repeated utilizing the recovered cells again according to the same manner as Example 3, 0.10 g of L-phenylalanine was produced.

Example 6

Incubation of culture and reaction were carried out in the same manner as Example 3, except that the strain was replaced by Pholiota highlandensis MCI 2037 (FERM P-8242). In the resulting reaction mixture, 0.10 g of L-phenylalanine was produced. Further, as the reaction was repeated utilizing the recovered cells again according to the same manner as Example 3, 0.07 g of L-phenylalanine was produced.

## Claims

1. A process for the preparation of L-phenylalanine comprising reacting cinnamic acid with ammonia in the presence of phenylalanine ammonia-lyase obtained from a microorganism and having an ability of producing L-phenylalanine from trans-cinnamic acid and ammonia, characterised in that said microorganism is a species of *Cladosporium, Pholiota, Hebeloma, Coprinus* or *Lyophyllum.*

2. A process as claimed in Claim 1, wherein the microorganism is selected from *Cladosporium cladosporiodies* C-5056 (FERM P-7704), *Pholiota highlandensis* MCI 2037 (FERM P-8242), *Hebeloma vinosophyllum* MCI 2038 (FERM P-8243), *Coprinus stercorarius* MCI 2035 (FERM P-8241) and *Lyophyllum tylicolor* MCI 2034 (FERM P-8240).

3. A process as claimed in Claim 1 or 2, wherein the reaction is carried out at a temperature of about 10 to 60°C.

4. A process as claimed in Claim 1, 2 or 3, wherein the reaction is carried out at a pH of 8 to 11.5.

5. A process as claimed in any of Claims 1 to 4, wherein the reaction is continued for a period of several hours to 4 days.

6. A process as claimed in any preceding claim, wherein the ammonia is aqueous ammonia or an ammonia precursor.

7. A process as claimed in Claim 6, wherein the ammonia precursor is an ammonium salt.

8. A process as claimed in Claim 7, wherein the ammonium salt is ammonium sulfate, ammonium chloride or ammonium acetate.

9. A process as claimed in any preceding claim wherein the reaction is carried out at a trans-cinnamic acid concentration of 0.1 to 5 w/v%.

10. A process as claimed in any preceding claim wherein the reaction is carried out at an ammonia concentration of 1 to 15 w/v%.

## Patentansprüche

1. Verfahren zur Herstellung von L-Phenylalanin, umfassend das Umsetzen von Zimtsäure mit Ammoniak in Gegenwart von Phenylalanin-Ammoniak-Lyase, die aus einem Mikroorganismus erhalten wurde und die Fähigkeit zur Herstellung von L-Phenylalanin aus trans-Zimtsäure und Ammoniak hat, dadurch gekennzeichnet, dass der Mikroorganismus ein Vertreter der Spezies Cladosporium, Pholiota, Hebeloma, Coorinus oder Lyophyllum ist.

2. Verfahren gemäss Anspruch 1, bei dem der Mikroorganismus aus Cladosporium cladosporiodies C-5056 (FERM P-7704), Pholiota highlandensis MCI 2037 (FERM P-8242), Hebeloma vinosophyllum MCI 2038 (FERM P-8243), Coprinus stercorarius MCI 2035 (FERM P-8241) und Lyophyllum tylicolor MCI 2034 (FERM P-8240) ausgewählt ist.

3. Verfahren gemäss Ansprüchen 1 oder 2, bei dem die Reaktion bei einer Temperatur von etwa 10 bis 60°C ausgeführt wird.

4. Verfahren gemäss Ansprüchen 1, 2 oder 3, bei dem die Reaktion bei einem pH-Wert von 8 bis 11,5 ausgeführt wird.

5. Verfahren gemäss Ansprüchen 1 bis 4, bei dem die Reaktion für einen Zeitraum von einigen Stunden bis 4 Tagen ausgeführt wird.

6. Verfahren gemäss einem der vorstehenden Ansprüche, bei dem Ammoniak wässriges Ammoniak oder ein Verläufer von Ammoniak ist.

7. Verfahren gemäss Anspruch 6, bei dem der Vorläufer von Ammoniak ein Ammoniumsalz ist.

8. Verfahren gemäss Anspruch 7, bei dem das Ammoniumsalz Ammoniumsulfat, Ammoniumchlorid oder Ammoniumacetat ist.

9. Verfahren gemäss einem der vorstehenden Ansprüche, bei dem die Reaktion bei einer Konzentration der trans-Zimtsäure von 0,1 bis 5 Gew./Vol.% ausgeführt wird.

10. Verfahren gemäss einem der vorstehenden Ansprüche, bei dem die Reaktion bei einer Ammoniakkonzentration von 1 bis 1,5 Gew./Vol.% ausgeführt wird.

## Revendications

1. Un procédé pour la préparation de L-phénylalanine consistant à faire réagir l'acide cinnamique et l'ammoniac en présence de phénylalanine-ammoniac-lyase obtenue à partir d'un micro-organisme et capable de produire la L-phénylalanine à partir d'acide trans-cinnamique et d'ammoniac, caractérisé en ce que ledit micro-organisme est une espèce du genre *Cladosporium, Pholiota, Hebeloma, Coprinus* ou *Lyophyllum.*

2. Un procédé selon la revendication 1, dans lequel le micro-organisme est choisi parmi *Cladosporium cladosporioides* C-5056 (FERM P-7704), *Pholiota highlandensis* MCI 2037 (FERM P-8242), *Hebeloma vinosophyllum* MCI 2038 (FERM P-8243), *Coprinus stercorarius* MCI 2035 (FERM P-8241) et *Lyophyllum tylicolor* MCI 2034 (FERM P-8240).

3. Un procédé selon la revendication 1 ou 2, dans lequel la réaction est mise en oeuvre à une température d'environ 10 à 60°C.

4. Un procédé selon la revendication 1, 2 ou 3, dans lequel la réaction est mise en oeuvre à un pH de 8 à 11,5.

5. Un procédé selon l'une quelconque des revendications 1 à 4, dans lequel la réaction est poursuivie pendant une durée de plusieurs heures à 4j.

6. Un procédé selon l'une quelconque des revendications précédentes, dans lequel l'ammoniac est de l'ammoniaque aqueuse ou un précurseur d'ammoniac.

7. Un procédé selon la revendication 6, dans lequel le précurseur d'ammoniac est un sel d'ammonium.

8. Un procédé selon la revendication 7, dans lequel le sel d'ammonium est le sulfate d'ammonium, le chlorure d'ammonium ou l'acétate d'ammonium.

9. Un procédé selon l'une quelconque des revendications précédentes, dans lequel la réaction est mise en oeuvre à une concentration d'acide trans-cinnamique de 0,1 à 5% en poids/volume.

10. Un procédé selon l'une quelconque des revendications précédentes, dans lequel la réaction est mise en oeuvre à une concentration d'ammoniac de 1 à 1,5% en poids/volume.